# EUROPEAN PATENT APPLICATION

(11) **EP 4 714 431 A1**
(43) Date of publication of application: **25.03.2026**
(21) Application number: 25203158.8
(22) Date of filing: 18.09.2025
(51) Int. Cl.: A61K 9/00, A61K 31/465, A61K 9/68, A61K 47/12, A61K 47/22, A61K 47/10, A61K 47/26, A61K 47/36, A61K 47/38, A61K 47/44, A61P 25/34

(54) **A NICTONE BASED GUM FORMULATION**

(30) Priority: 19.09.2024 EP 24201324
(71) Applicant: Mehta, Bharat, Sultanpur New Delhi 110030 (IN)
(72) Inventor: Mehta, Bharat, Sultanpur New Delhi 110030 (IN)
(74) Representative: HGF

(57) **Abstract**

The present invention is related to a nicotine-based gum formulation. The present invention is particularly related to a nicotine-based gum formulation comprising nicotine, a gelling agent, plasticizer, release control agent, acidifier, acid regulating agent, flavouring agent, sweetener, thickening agent and colouring agent. The present invention also relates to the process for preparing the nicotine-based gum formulation.

## Description

### FIELD OF THE INVENTION

The present invention relates to a gum formulation containing nicotine. More particularly, the present invention relates to a soft gum formulation comprising nicotine, which can effectively deliver nicotine at a low pH-value. The present invention also relates to the process for preparation of the nicotine-based gum formulation. The disclosed chewing-gum formulation serves as a nicotine replacement therapy, aiming to minimize common side effects such as hiccups, headaches, nausea, throat irritation and burning sensations.

### BACKGROUND OF THE INVENTION

Smoking behaviour is associated with serious health risks not only to the smoker, but also to the people around them exposed to passive smoke. Thus, experts from many years have advised to quit smoking. However, the smoker's addiction to nicotine, makes it difficult for most smokers to quit. Many ways of nicotine administration have been employed in the efforts to help smokers quit their unhealthy habit. Several products employing oral or transdermal administration of nicotine are currently available in the market for smokers wanting to quit smoking such as chewing gums, inhalers, patches or mouth sprays.

As tobacco contains numerous toxic compounds in addition to nicotine, nicotine substitution products are also relevant for individuals who consume their tobacco in other ways than by smoking. The main problem with tobacco smoking is its enormous implications on health. Today it is estimated that use of tobacco causes more than 8 million deaths per year. Even though today tobacco smoking is decreasing in many developed countries today there is a continuous need of creating awareness for quitting this unhealthy habit.

Nicotine is an addictive alkaloid C₁₀H₁₄N₂, derived from the tobacco plant. It is an organic compound and is the principal alkaloid of tobacco. Nicotine is the main addictive ingredient in the tobacco used in cigarettes, cigars, snuff and other nicotine-containing products. Nicotine is the world's second most used drug, after caffeine from coffee and tea. It is very addictive and thus characteristically display a strong tendency to relapse after having successfully abstained from smoking for a time. The administration of nicotine can provide satisfaction. When smoking a cigarette, nicotine is quickly absorbed into the smoker's blood and reaches the brain in around ten seconds after inhalation. The quick uptake of nicotine gives the consumer a rapid satisfaction, or a kick. The satisfaction of smoking a cigarette lasts during the smoking time and for a period of time thereafter. The poisonous, toxic, carcinogenic, and addictive nature of smoking has initiated efforts to search for methods, compositions and devices, which would help in breaking the habit of smoking cigarettes.

Several methods and means have been described for reducing the desire of a subject to use tobacco, which comprises the step of administering to the subject nicotine or a derivative thereof as described in few of the patent applications. The applications are US4967773A (Nicotine containing lozenge), US20240000713 (Water-soluble nicotine tablet), GB2299756 (Pastilles containing nicotine), US5810018A (oral nicotine spray), US20100004294 (Stable lozenge containing nicotine).

Thus, there are prior arts available for nicotine containing various formulations for reducing the nicotine addiction. The major formulation available in the market is a gum base formulation or solid formulation like lozenges. But there are various problems in the prior art composition as nicotine is retained in the gum due to incomplete chewing resulting in low systemic bioavailability. The chewing of gums or lozenges mentioned in the prior art leads to all of a sudden release of nicotine in buccal cavity which in turn causes nausea and burning sensation. Moreover, accidental swallowing results in sudden release of nicotine in GI tract causing nausea and burning sensation.

Soft gum products with a pH of 5.0 or higher tend to be less enjoyable to consume due to a pronounced nicotine release, which can lead to strong side effects such as hiccups, headaches, nausea, and a burning throat. In contrast, gums with a pH below 5.0 experience slow nicotine release and fewer side effects. Specifically, gums with a pH ranging from 3.5 to 3.9 not only lower the side effects but also improve the masking of both nicotine and the product's taste. This improvement is particularly noticeable in gums with natural plant flavors like peppermint, orange-lemon, and blueberry. For gums with more neutral flavors, such as those mimicking dried or processed plant parts like coffee and cinnamon, the best masking of nicotine occurs at a pH between 4.0 and 4.5.

Though there are various nicotine containing formulations available in the market, there is a need to develop a formulation overcoming all the shortcomings of the available formulations. Therefore, the inventors of the present invention have provided a formulation that maximizes buccal absorption of nicotine. Also, the present formulation overcomes the associated symptoms of nicotine release such as nausea, hiccups or burning sensation. Also, the oral formulation of the present invention provides release of nicotine in a controlled manner over a prolonged period of time without unpleasant mouth feel and taste.

The objectives of the present invention are as described herein.

### OBJECTIVE OF THE INVENTION

The main objective of the invention is to provide a nicotine-based gum formulation.

The other main objective of the invention is to provide a nicotine-based gum formulation that maximizes buccal absorption of nicotine.

Another objective of the present invention is to provide a prolonged release of nicotine in a controlled manner without unpleasant taste and mouth feel.

Yet another objective of the present invention is to provide the formulation of nicotine that prevents symptoms such as nausea, hiccups or burning sensation.

Another objective of the invention is to provide a nicotine-based gum formulation which has a better consumer compliance and is suitable for consumers with chewing or swallowing difficulties.

Yet another objective of the present invention is to provide a nicotine-based gum formulation that is gentle on teeth, gums and the oral cavity.

### SUMMARY OF THE INVENTION

The main aspect of the present invention is to provide a nicotine-based gum formulation.

Another main aspect of the present invention is to provide a nicotine-based gum formulation comprising nicotine, a gelling agent, plasticizer, release control agent, acidifier, acid regulating agent, and one or more pharmaceutical excipients.

The other aspect of the present invention is to provide a nicotine-based gum formulation comprising nicotine, a gelling agent, plasticizer, release control agent, acidifier, acid regulating agent, flavouring agent, sweetener, thickening agent and colouring agent.

Another aspect of the invention is to provide a nicotine-based gum formulation and process of preparation of the same.

The details of one or more aspect of the invention are set forth in the description below. Other features, objects and advantages of the invention will be apparent from the description.

### DESCRIPTION OF THE INVENTION

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described.

As used herein, the singular forms "a," "an" and "the" specifically also encompass the plural forms of the terms to which they refer, unless the content clearly dictates otherwise.

As used herein, the term "formulation" or "composition", unless otherwise defined refers to gum includes soft gum, pastilles, soft pastilles, chewing pastilles, as well as gelled, dimensionally stable soft gel products and hard gum products.

As mentioned herein the body of the invention the "gum formulation" includes soft gum, pastilles, soft pastilles, chewing pastilles, as well as gelled, dimensionally stable soft gel products and hard gum products.

As used herein, the terms "releasing agent" and "release control agents" have the same meaning and can be used interchangeably.

The term "chewing gum" is a novel drug delivery system containing gum base with pharmacologically active ingredient and intended to use for systemic absorption through oral mucosa.

The main embodiment of the present invention to provide a nicotine-based gum formulation.

Another main embodiment of the present invention is to provide a nicotine-based gum formulation comprising nicotine, a gelling agent, plasticizer, release control agent, acidifier, acid regulating agent, and one or more pharmaceutical excipients.

Another embodiment of the present invention is to provide a nicotine-based gum formulation comprising nicotine, a gelling agent, plasticizer, release control agent, acidifier, acid regulating agent, flavouring agent, sweetener, thickening agent and colouring agent.

As per one another embodiment of the present invention, the said nicotine is selected from group consisting of nicotine polacrilex, nicotine resinate and nicotine salts such as nicotine benzoate, nicotine salicylate, nicotine lactate, nicotine levulinate and combinations thereof.

As per a preferred embodiment, the nicotine is nicotine polacrilex.

As per another embodiment of the present invention, the said nicotine is used in the range of 0.1 mg to 20 mg, more preferably in the range of 0.5 mg to 15 mg and most preferably in the range of 1 mg to 10 mg.

As per another embodiment of the present invention, the said gelling agent is selected from tragacanth, pectin, starch, carbomer, sodium alginate, gelatin, cellulose derivatives, and polyvinyl alcohol clays.

As per a preferred embodiment, the gelling agent is gelatin.

As per another embodiment of the present invention, the said gelling agent is used in the range of 1 mg to 500 mg, more preferably 50 mg to 450 mg and most preferably 100 mg to 350 mg.

As per another embodiment of the present invention, the said plasticizer is selected from vegetable oils, hydrogenated vegetable oils, triacetin, glycerin, propylene glycol.

As per a preferred embodiment, the plasticizer is glycerin.

As per another embodiment of the present invention, the said plasticizer is used in the range of 100 mg to 1000 mg, more preferably 400 mg to 800 mg and most preferably 600 mg to 750 mg.

As per another embodiment of the present invention, the ratio of gelling agent to plasticizer for the preparation of soft gum containing is in the range of about 1:2 to about 1:5.

As per another embodiment of the present invention, the said release control agent is selected from oil, lecithin, lanolin, mono-glycerides and di-glycerides, stearic acid, sodium stearate, glycerine, potassium stearate and glycerol triacetate.

As per a preferred embodiment, the said release control agent is lecithin.

As per another embodiment of the present invention, the said release control agent is used in the range of 1 mg to 200 mg, more preferably 20 mg to 150 mg and most preferably 50 mg to 100 mg.

As per another embodiment of the present invention, wherein the said acidifiers are used for anti-oxidation and masking the taste of nicotine, supporting better taste and decreasing the pH-value.

As per another embodiment of the present invention, the said acidifier is selected from group consisting of ascorbic acid, calcium ascorbate, citric acid and malic acid and combinations thereof.

As per a preferred embodiment, the acidifier is a combination of ascorbic acid and citric acid.

As per another embodiment of the present invention, the said acid regulating agent is used in the range of 0.1 mg to 50 mg, more preferably 1 mg to 15 mg and most preferably 2 mg to 10 mg.

As per another embodiment of the present invention, the said acid regulating agent is used in the nicotine base gum formulation as a buffer to adjust the pH-value of the gum.

As per another embodiment of the present invention, the said acid regulating agent is selected from group consisting of sodium salts of water-soluble organic acids.

As per a preferred embodiment, the acid regulating agent is sodium citrate.

As per another embodiment of the present invention, the said acid regulating agent is used in the range of 0.1 mg to 20 mg, more preferably 1 mg to 15 mg and most preferably 2 mg to 10 mg.

As per another embodiment of the present invention, the said formulation comprises of one or more pharmaceutically acceptable excipients selected from flavouring agent, sweetener, thickening agents, colouring agent or combinations thereof.

As per another embodiment of the present invention, the said flavouring agent is selected from natural and / or artificial flavours, including-but not limited to-menthol, vanillin, cinnamon, peppermint, lemon, mint, coffee, strawberry, banana, pineapple, orange, raspberry, and combinations thereof.

As per another embodiment of the present invention, the said flavouring agent is used in the range of 1 mg to 100 mg, more preferably 5 mg to 60 mg and most preferably 10 mg to 50 mg.

As per one embodiment of the present invention, sweetener can be selected from the group consisting of stevia, sucralose and sucrose and combinations thereof.

As per one embodiment of the present invention, the said sweetener is used in the range of 0.1 mg to 50 mg, more preferably 0.5 mg to 25 mg and most preferably 1 mg to 10 mg.

As per another embodiment of the present invention, the thickening agent is selected from guar gum, locust bean gum, inulin, gum arabic and carrageenan and combinations thereof.

As per a preferred embodiment, the thickening agent is a combination of guar gum and inulin.

As per another embodiment, the said thickening agent is used in the range of 0.1 mg to 50 mg, more preferably 0.5 mg to 45 mg and most preferably 1 mg to 35 mg.

As per another embodiment of the present invention, the colouring agent can be selected from group consisting of plant extracts or natural colours.

As per another embodiment of the present invention, the said nicotine-based gum formulation comprises water about 5% to about 15% of the mass of the soft gum.

As per another embodiment of the present invention, the nicotine-based gum formulation are free of preservatives.

As per one embodiment of the present invention, the pH of the nicotine-based gum formulation is determined at 20-30 deg C by using a pH meter by dissolving and sonicating the 1200mg of gum for 10 to 20 minutes in 20 ml HPLC water.

As per one embodiment of the present invention, the pH-value of the soft gum is below pH 5.

As per one embodiment of the present invention, the pH-value of the soft gum in the range of pH 3.0 to pH 4.8.

As per one another embodiment of the present invention, said nicotine soft gum is capable of being dissolved in the buccal cavity in about 5 to about 15 minutes, depending on the user's sucking pattern.

As per another embodiment of the present invention, the process for preparation of nicotine gum-based formulation comprises following steps:
a. Weighing plasticizer and water in a reactor;
b. Adding gelling agent and release control agent in mixture of step (a);
c. Adding nicotine active in the mixture of step (b);
d. Adding adequate quantities of sweetener, acidifiers, acid regulating agents, thickening agents, flavouring agents and colouring agents in the mixture of step (c) obtaining a mass;
e. Collecting the mass of step (d) in a container followed by cooling and solidification;
f. Transferring the solidified mass of step of step (e) into a melter obtaining melted mass; and
g. Passing the melted mass through an injector into the preformed cavities followed by cooling and blister packaging.

As per another embodiment of the present invention, the said nicotine based gum formulation comprises 0.1 mg to 20 mg of nicotine, 1 mg to 500 mg of gelling agent, 100 mg to 1000 mg of plasticizer, 1 mg to 200 mg of release control agent, 0.1 mg to 50 mg of acidifier, 0.1 mg to 20 mg of acid regulating agent, 1 mg to 100 mg of flavouring agent, 0.1 mg to 50 mg of sweetener, 0.1 mg to 50 mg of thickening agent, 0.1 mg to 20 mg of colouring agent and water.

As per another embodiment of the present invention, the said nicotine-based gum formulation comprises 3.5 mg of nicotine, 241.73 mg of gelling agent, 627.28 mg of plasticizer, 80.0 mg of release control agent, 40 mg of acidifier, 5 mg of acid regulating agent, 2 mg of flavouring agent, 4.80 mg of sweetener, 20 mg and 5 mg of thickening agent, 5 mg of colouring agent and water.

As per another embodiment of the present invention, the said nicotine-based gum formulation can be soft gum, pastilles, soft pastilles, chewing pastilles, as well as gelled, dimensionally stable soft gel products and hard gum products.

As per another embodiment of the present invention, the said nicotine gum base formulation helps in reducing the side effects of nicotine like hiccups, headaches, nausea and burning sensation of the throat.

As per another embodiment of the present invention, the said nicotine gum base formulation generates a pleasant taste and flavour release overcoming the unpleasant experience of consuming nicotine.

As per another embodiment of the present invention, the said nicotine gum base formulation is used as a nicotine replacement product for smokers or vapours.

The invention is further illustrated by the following examples, which are provided to be exemplary of the invention and do not limit the scope of the invention. While the present invention has been described in terms of its specific embodiments, certain modifications and equivalents will be apparent to those skilled in the art and are intended to be included within the scope of the present invention.

The invention is also defined by the following numbered clauses:
1. A nicotine based gum formulation comprising nicotine, a gelling agent, plasticizer, releasing agent, acidifier, acid regulating agent, and one or more pharmaceutical excipients.
2. The nicotine based gum formulation of clause 1 comprising nicotine in an amount of about 0.1 - 0.2 wt% , a gelling agent in an amount of about 20 wt%, plasticizers in an amount of about 50 wt%, releasing agent in an amount of about 6 - 7 wt%, acidifier in an amount of about 3-4 wt%, acid regulating agent in an amount of about 0.35-0.45 wt%, and one or more pharmaceutical excipients.
3. The nicotine based gum formulation of clause 1 or clause 2, wherein the said nicotine is selected from group consisting of nicotine polacrilex, nicotine resinate and nicotine lactate and combinations thereof.
4. The nicotine based gum formulation of any preceding clause, wherein the said gelling agent is selected from tragacanth, pectin, starch, carbomer, sodium alginate, gelatin, cellulose derivatives, and polyvinyl alcohol clays.
5. The nicotine based gum formulation of in any preceding clause, wherein the said plasticizer is selected from lecithin, lanolin, glycerides, stearic acid, sodium stearate, glycerine, potassium stearate and glycerol triacetate.
6. The nicotine based gum formulation of any preceding clause, wherein the said releasing agent is selected from oil, lecithin, lanolin, mono-glycerides and di-glycerides, stearic acid, sodium stearate, glycerine, potassium stearate and glycerol triacetate.
7. The nicotine based gum formulation of any preceding clause, wherein the said acidifier is selected from group consisting of ascorbic acid, calcium ascorbate, citric acid and malic acid and combinations thereof.
8. The nicotine based gum formulation of any preceding clause, wherein the said acid regulating agent is selected from group consisting of sodium salts of water-soluble organic acids.
9. The nicotine based gum formulation of any preceding clause, wherein the said one or more pharmaceutical excipients can be selected from flavouring agent, sweetener, thickening agent, colouring agent or combinations thereof.
10. The the nicotine based gum formulation of clause 1, wherein the formulation comprises nicotine, gelatin, glycerin, lecithin, ascorbic acid, citric acid, trisodium citrate, and one or more pharmaceutical excipients.
11. The nicotine based gum formulation of any preceding clause for use in the treatment of nicotine dependence.
12. A process for preparing a nicotine based gum formulation, the process comprising the following steps:
   a) Weighing plasticizer and water in a reactor;
   b) Adding gelling agent and releasing agent in mixture of step (a);
   c) Adding nicotine active in the mixture of step (b);
   d) Adding adequate quantities of sweetener, acidifiers, acid regulating agent, thickening agents, flavouring agents and colouring agents in the mixture of step (c) obtaining a mass;
   e) Collecting the mass of step (d) in a container followed by cooling and solidification;
   f) Transferring the solidified mass of step of step (e) into a melter to obtain melted mass;
   g) Passing the melted mass of step (f) through an injector into the preformed cavities followed by cooling and blister packaging.

### EXAMPLES

### EXAMPLE 1: TRIAL BATCHES FOR OPTIMIZING THE COMPOSITION OF NICOTINE CHEWING GUM

The objective of this was to optimize the composition of the nicotine chewing gum. The below provided are the three trials taken for the optimization of the composition of the nicotine-based chewing gum.

**Table 1: Trials batches for the optimization of nicotine gum formulation**

| **SR. NO.** | **INGREDIENTS** | **Formulation 1 (in mg)** | **Formulation 2 (in mg)** | **Formulation 6 (in mg)** |
|---|---|---|---|---|
| 1. | Nicotine from Nicotine polacrilex | 2.0 | 3.5 | 2.0 |
| 2. | Gelatin (bloom strength 250) | 243.75 | 241.73 | 206.10 |
| 3. | Glycerin | 632.53 | 627.28 | 713.11 |
| 4. | Water | 98.72 | 97.90 | 111.29 |
| 5. | Lecithin | 80.0 | 80.0 | 80.0 |
| 6. | Ascorbic acid | 20.00 | 20.00 | 20.00 |
| 7. | Citric acid | 20.00 | 20.00 | - |
| 8. | Tri-sodium citrate | 5.00 | 5.00 | 4.00 |
| 9. | Sucralose | 4.50 | 4.80 | 4.50 |
| 10. | Steviol glycosides | 3.50 | 3.80 | 3.50 |
| 11. | Menthol | 2.00 | 2.00 | - |
| 12. | Plant extract (for colouring) | 5.00 | 5.00 | 12.00 |
| 13. | Inulin | 20.00 | 20.00 | 3.00 |
| 14. | Guar gum | 5.00 | 5.00 | 1.5 |
| 15. | Flavor (flavor oils) | 20.00 | 20.00 | - |
| 16. | Flavor | 30.00 | 30.00 | 35.00 |

### Procedure:

a) Weighing glycerin and water in a reactor;
b) Adding gelatin and lecithin in mixture of step (a);
c) Adding nicotine active in the mixture of step (b);
d) Adding adequate quantities of sucralose, ascorbic acid, citric acid, Cirtic acid, Tri-sodium citrate, guar gum, inulin, flavouring agents and colouring agents in the mixture of step (c) obtaining a mass;
e) Collecting the mass of step (d) in a container followed by cooling and solidification;
f) Transferring the solidified mass of step of step (e) into a melter to obtain melted mass;
g) Passing the melted mass of step (f) through an injector into the preformed cavities followed by cooling and blister packaging.

### EXAMPLE 2: ORGANOLEPTIC TESTING OF NICOTINE CHEWING GUM

The study was carried out to compare standard mint formulations (2 mg and 4mg) with present invention mint flavoured Formulation 1 (Nicotine 2 mg), Formulation 2 (Nicotine 3.5 mg) and Formulation 6 (Nicotine 2 mg) using sensory-based user feedback. The goal is to assess user experience in terms of comfort, taste, irritation, and nicotine effect release.

### Test Description:

• Each sample was evaluated based on **10 sensory parameters.**
• Ratings were collected from **trained panellists** and converted into **band scores** for simplification.
• **Session-wise details:**
∘ **Session 1 (2 mg comparison):** 10 testers
∘ **Session 2 (3.5 mg comparison):** 10 testers
∘ **Session 6 (2 mg comparison):** 30 testers
• **Band Score Conversion Logic:**

| **Average Score** | **Band Score** | **Interpretation** |
|---|---|---|
| **1.0 - 1.5** | A | Best (most desirable) |
| **1.5 - 2.0** | B | Acceptable |
| > **2.0** | C | Least preferred |

### Results:

### Session 1: Standard Mint (2 mg) vs Formulation 1 (2 mg)

### Number of testers: 10

**Table 2: Result for session 1 of the nicotine gum formulation**

| **Parameter** | **Standard Mint (2 mg)** | **Formulation 1 (2 mg)** | **Comparison Outcome** |
|---|---|---|---|
| **Time required to feel release of Nicotine** | **A** | **A** | Equal |
| **Stomach Irritation** | **A** | **A** | Equal |
| **Pain in jaws** | **A** | **A** | Equal |
| **Feeling of Nausea and vomiting** | **A** | **A** | Equal |
| **Taste** | **C** | **B** | Formulation 1 Better |
| **Smoothness** | **C** | **B** | Formulation 1 Better |
| **Burning Sensation in buccal cavity** | **B** | **A** | Formulation 1 Better |
| **Burning Sensation in throat** | **C** | **A** | Formulation 1 Better |
| **Hiccups** | **A** | **A** | Equal |
| **Flavour** | **C** | **B** | Formulation 1 Better |

**Conclusion of Session 1:** Formulation 1 shows **equal performance in 5 out of 10 parameters** (mostly core health-effect ones) and **improves in 5 other comfort and sensory parameters** like taste, smoothness, and reduced burning sensations. Formulation 1 is a superior alternative to Standard Mint (2 mg), especially in enhancing mouthfeel and overall experience.

### Session 2: Standard Mint (4 mg) vs Formulation 2 (3.5 mg)

### Number of testers: 10

**Table 3: Result for session 2 of the nicotine gum formulation**

| **Parameter** | **Standard Mint (4 mg)** | **Formulation 2 (3.5 mg)** | **Comparison Outcome** |
|---|---|---|---|
| **Time required to feel release of Nicotine** | B | A | Formulation 2 Better |
| **Stomach Irritation** | B | A | Formulation 2 Better |
| **Pain in jaws** | B | A | Formulation 2 Better |
| **Feeling of Nausea and vomiting** | A | A | Equal |
| **Taste** | C | A | Formulation 2 Better |
| **Smoothness** | C | B | Formulation 2 Better |
| **Burning Sensation in buccal cavity** | C | A | Formulation 2 Better |
| **Burning Sensation in throat** | C | A | Formulation 2 Better |
| **Hiccups** | C | A | Formulation 2 Better |
| **Flavour** | C | A | Formulation 2 Better |

**Conclusion of Session 2:** Formulation 2 exhibits superior performance in 9 of 10 parameters, even with 0.5 mg lower nicotine content. It matches Standard Mint (4 mg) in nausea control and excels in comfort metrics like taste, smoothness, and lower irritation. Formulation 2 is a significantly improved version over Standard Mint (4 mg), offering better user tolerance and experience.

### Session 6: Standard Mint (2 mg) vs Formulation 6 (2 mg)

### Number of testers: 30

**Table 3: Result for session 6 of the nicotine gum formulation**

| **Parameter** | **Standard Mint (2 mg)** | **Formulation 6 (2 mg)** | **Comparison Outcome** |
|---|---|---|---|
| **Time required to feel release of Nicotine** | B | A | Formulation 6 Better |
| **Stomach Irritation** | B | A | Formulation 6 Better |
| **Pain in jaws** | A | A | Equal |
| **Feeling of Nausea and vomiting** | B | A | Formulation 6 Better |
| **Taste** | C | B | Formulation 6 Better |
| **Smoothness** | C | A | Formulation 6 Better |
| **Burning Sensation in buccal cavity** | B | A | Formulation 6 Better |
| **Burning Sensation in throat** | C | A | Formulation 6 Better |
| **Hiccups** | A | A | Equal |
| **Flavour** | C | B | Formulation 6 Better |

**Conclusion of Session 6:** Formulation 6 performs equal in 2 parameters (Pain in jaws and Hiccups) and better in all remaining 8 parameters, especially where smoother feel, faster release, and reduced irritation are desired. Formulation 6 clearly outperforms Standard Mint (2 mg), as validated by a larger sample size (30 testers).

### Final Comparative Summary Table:

**Table 4: Final comparative analysis of all sessions for the nicotine gum formulation**

| **Formulation** | **Reference Compared Against** | **No. of Testers** | **Equal Parameters** | **Better Parameters** | **Inferior Parameters** | **Verdict** |
|---|---|---|---|---|---|---|
| **Formulation 1** | Standard Mint (2 mg) | 10 | 5 | 5 | 0 | Superior to Standard Mint (2 mg) |
| **Formulation 2** | Standard Mint (4 mg) | 10 | 1 | 9 | 0 | Much better than 4 mg version |
| **Formulation 6** | Standard Mint (2 mg) | 30 | 2 | 8 | 0 | Best performing vs 2 mg standard |

### Conclusion of the study:

- All formulations **perform equal or better** than their respective standard references.
- **No formulation is inferior** in any parameter.
- The data supports transitioning toward Formulations 1, 2, and 6 for **enhanced user satisfaction, reduced side effects,** and better flavour/mouthfeel experience.
- Especially with **Formulation 2,** better performance is achieved even with **lower nicotine content,** making it efficient and user-friendly.

### EXAMPLE 3: TRIALS FOR USER EXPERIENCE FOR DIFFERENT FLAVOURS

The objective of this study was to evaluate different flavours components on user experience when the nicotine content and base formulation is kept constant. The study helps for designing the flavour on sensory comfort parameters such as taste, smoothness, burning sensations, and overall flavour perception.

**Testing:** Four different formulation containing 3.5 mg of nicotine was carried out. Further a panel of trained testers rated each sample on 10 sensory parameters. The first 4 physiological parameters (e.g., time to feel nicotine, irritation, jaw pain, nausea) remained unchanged across samples due to consistent formulation. The remaining 6 parameters were impacted by the flavouring components used.

**Table 5: Result for different flavour formulation of the nicotine gum formulation**

| **Formulation** | **Taste** | **Smoothness** | **Burning in Throat** | **Flavour** | **Time to Nicotine Release** | **Stomach Irritation** | **Pain in Jaws** | **Nausea & Vomiting** | **Burning in Buccal Cavity** | **Hiccups** |
|---|---|---|---|---|---|---|---|---|---|---|
| Formulation 2 (Mint flavour) | A | B | A | A | A | A | A | A | A | A |
| Formulation 3 (coffee flavour) | C | B | A | B | A | A | A | A | A | A |
| Formulation 4 (orange/lemon flavour) | B | A | B | A | A | A | A | A | A | A |
| Formulation 5 (Blueberry flavour) | B | B | A | B | A | A | A | A | A | A |

**Conclusion:** Formulation 2 is the top performer, with only one 'B' score (Smoothness) and 'A' ratings in all other parameters, making it the most preferred flavour from a sensory standpoint. Formulation 4 comes in second, with two B scores in Taste and Burning Sensation in the Throat, and performs well in all other parameters. Formulations 3 and 5 show identical performance, receiving three lower scores (Taste, Smoothness, Flavour), indicating a need for improvement in flavour composition to enhance user acceptability. For future development, Formulation 2 may serve as the benchmark flavour profile for replication or enhancement.

## Claims

1. A nicotine-based gum formulation, wherein said formulation comprises of nicotine, a gelling agent, plasticizer, release control agent, acidifier, acid regulating agent, flavouring agent, sweetener, thickening agent and colouring agent.

2. The nicotine base gum formulation as claimed in claim 1, wherein said nicotine is selected from group consisting of nicotine polacrilex, nicotine resinate and nicotine salts such as nicotine benzoate, nicotine salicylate, nicotine lactate, nicotine levulinate and combinations thereof.

3. The nicotine-based gum formulation as claimed in claim 1, wherein said nicotine is used in the range of 0.1 mg to 20 mg, more preferably in the range of 0.5 mg to 15 mg and most preferably in the range of 1 mg to 10 mg.

4. The nicotine-based gum formulation as claimed in claim 1, wherein said gelling agent is selected from tragacanth, pectin, starch, carbomer, sodium alginate, gelatin, cellulose derivatives, and polyvinyl alcohol clays.

5. The nicotine-based gum formulation as claimed in claim 1, wherein said gelling agent is used in the range of 1 mg to 500 mg, more preferably 50 mg to 450 mg and most preferably 100 mg to 350 mg.

6. The nicotine-based gum formulation as claimed in claim 1, wherein said plasticizer is selected from vegetable oils, hydrogenated vegetable oils, triacetin, glycerin, propylene glycol.

7. The nicotine-based gum formulation as claimed in claim 1, wherein said plasticizer is used in the range of 100 mg to 1000 mg, more preferably 400 mg to 800 mg and most preferably 600 mg to 750 mg.

8. The nicotine-based gum formulation as claimed in claim 1, wherein the gelling agent to plasticizer is in the range of about 1:2 to about 1:5.

9. The nicotine-based gum formulation as claimed in claim 1, wherein said release control agent is selected from oil, lecithin, lanolin, mono-glycerides and di-glycerides, stearic acid, sodium stearate, glycerine, potassium stearate and glycerol triacetate.

10. The nicotine-based gum formulation as claimed in claim 1, wherein said release control agent is used in the range of 1 mg to 200 mg, more preferably 20 mg to 150 mg and most preferably 50 mg to 100 mg.

11. The nicotine-based gum formulation as claimed in claim 1, wherein said acidifier are selected from group consisting of ascorbic acid, calcium ascorbate, citric acid and malic acid and combinations thereof.

12. The nicotine-based gum formulation as claimed in claim 1, wherein said acidifier is used in the range of 0.1 mg to 50 mg, more preferably in the range of 5 mg to 30 mg and most preferably in the range of 10 mg to 25 mg.

13. The nicotine-based gum formulation as claimed in claim 1, wherein said acid regulating agent is selected from group consisting of sodium salts of water-soluble organic acids.

14. The nicotine-based gum formulation as claimed in claim 1, wherein said acid regulating agent is used in the range of 0.1 mg to 20 mg, more preferably 1 mg to 15 mg and most preferably 2 mg to 10 mg.

15. The nicotine-based gum formulation as claimed in claim 1, wherein said formulation comprises of one or more pharmaceutically acceptable excipients selected from flavouring agent, sweetener, thickening agents, colouring agent or combinations thereof.

16. The nicotine-based gum formulation as claimed in claim 1, wherein said thickening agent is selected from guar gum, locust bean gum, inulin, gum arabic and carrageenan and combinations thereof

17. The nicotine-based gum formulation as claimed in claim 1, wherein process for preparation of nicotine gum-based formulation comprises following steps:
a) Weighing plasticizer and water in a reactor;
b) Adding gelling agent and release control agent in mixture of step (a);
c) Adding nicotine active in the mixture of step (b);
d) Adding adequate quantities of sweetener, acidifiers, acid regulating agents, thickening agents, flavouring agents and colouring agents in the mixture of step (c) obtaining a mass;
e) Collecting the mass of step (d) in a container followed by cooling and solidification;
f) Transferring the solidified mass of step of step (e) into a melter obtaining melted mass; and
g) Passing the melted mass through an injector into the preformed cavities followed by cooling and blister packaging.

18. The nicotine based gum formulation as claimed in claim 1, wherein said nicotine based gum formulation comprises 0.1 mg to 20 mg of nicotine, 1 mg to 500 mg of gelling agent, 100 mg to 1000 mg of plasticizer, 1 mg to 200 mg of release control agent, 0.1 mg to 50 mg of acidifier, 0.1 mg to 20 mg of acid regulating agent, 1 mg to 100 mg of flavouring agent, 0.1 mg to 50 mg of sweetener, 0.1 mg to 50 mg of thickening agent, 0.1 mg to 20 mg of colouring agent and water.

19. The nicotine-based gum formulation as claimed in claim 1, wherein said soft gum has a low pH-value below pH 5.
